# EUROPEAN PATENT APPLICATION

(11) **EP 4 400 153 A1**
(43) Date of publication of application: **17.07.2024**
(21) Application number: 23305046.7
(22) Date of filing: 13.01.2023
(51) Int. Cl.: A61M 60/178, A61M 60/857

(54) **MEMBRANE CROSSING LEFT CARDIAC ASSISTING DEVICE**

(71) Applicant: HTC-Assistance, 45100 Orléans (FR)
(72) Inventor: NICOL, Aurélien, 45100 Orléans (FR); BREDENBREUKER, Lars, 45100 Orléans (FR); HELLUY, Océane-Aurore, 45100 Orléans (FR)
(74) Representative: Icosa

(57) **Abstract**

The present invention relates to a left cardiac assisting device (10) to be implanted inside a patient's heart (100), comprising:
- an inlet conduct (12) configured to be secured to a first membrane of the patient's heart (100),
- an outlet conduct (14) configured to be secured to a second membrane separating either the right atrium from the aorta,
- a pump housing (16) configured to surround a blood pump,
- a removable pump support element configured to cooperate with the inlet conduct.

The inlet conduct, the outlet conduct and the pump housing form one single technical piece. The inlet conduct, the outlet conduct and the pump housing are configured to expand independently from each other. The removable pump support element guides the inlet conduct through the first membrane during implantation.

## Description

### FIELD OF INVENTION

The present invention relates to a percutaneous implantable heart assisting device for the left side of the heart.

### BACKGROUND OF INVENTION

Heart failure remains a major health problem, with an estimated prevalence of 1-2% in the adult population of developed countries increasing to 10% from the age of 70 years.

Given the size of the long-term cardiac assisting devices currently known in the state of the art, it is technically not possible to implant them percutaneously. Nor is it possible to implant two, one in the left ventricle, the other in the right ventricle in case of double failure for patients not eligible for a heart transplant.

Considering the human anatomy, it appears that the best location for percutaneous assisting device to be implanted, is within the ventricles of the heart. However, the long term left ventricle assisting devices known in the state of the art are usually too large to be folded and therefore cannot be implanted inside the ventricle from the arterial access. Assisting devices cannot be miniaturized below a certain size in order to remain effective in moving the average human blood flow.

The technical problem to be solved by the present invention is thus to provide an effective long-term left ventricular assisting device that can be safely percutaneously implanted inside any human heart, by the femoral vein, with minimally invasive surgical techniques avoiding any cardiopulmonary bypass. The device according to the present invention can thus be implanted without opening the patient's heart, in a mini-invasive way.

### SUMMARY

The present invention aims at solving this problem and thus relates to a left cardiac assisting device configured to be implanted inside a patient's heart, said assisting device comprising:
- an inlet conduct configured to be secured to a first membrane of the patient's heart,
- an outlet conduct configured to be secured to a second membrane of the patient's heart, the second membrane separating either the right atrium from the aorta or the superior vena cava from the aorta,
- a pump housing configured to surround a blood pump, said pump housing connecting the inlet conduct to the outlet conduct, the pump housing being further designed to be located inside the right atrium or the superior vena cava of the patient's heart,
- a removable pump support element configured to cooperate with the inlet conduct. In the left cardiac assisting device according to the present invention:
- the inlet conduct, the outlet conduct and the pump housing form one single technical piece, in order to enable the patient's blood flow to be driven from the inlet conduct to the outlet conduct through the first and second membranes the patient's heart,
- the inlet conduct, the outlet conduct and the pump housing are configured to expand independently from each other,
- the removable pump support element is configured to enable the guiding of the inlet conduct through the first membrane of the patient's heart implantation.

Thus, this solution achieves the above objective. In particular, it allows the obtaining of a fully percutaneous long-term left ventricular assistance through the veinous femoral access. The device according to the present invention further enables to implant patients with narrow atriums as the specific shape of the assisting device according to the present invention is adapted to any patient's heart.

The device according to the invention may include one or more of the following characteristics, taken in isolation from one another or in combination with one another:
- the assisting device may be configured to extend along the long heart axis, the inlet conduct being thus configured to be located lower than the outlet conduct regarding said long heart axis, inside the patient's heart,
- the outlet conduct may be configured to extend transversally to the long heart axis, inside the patient's heart,
- the assisting device may present a general Y or T shape,
- the removable pump support element can be bent in order to present a general L shape and is configured to be removably secured to the first membrane of the patient's heart,
- the first membrane may be the membrane separating the left atrium from the right atrium of the patient's heart, or the membrane separating the left atrium from the superior vena cava from the patient's heart,
- the inlet conduct may be configured to cooperate radially with an edge of a first anastomosis aperture inside the first membrane, and the outlet conduct is configured to cooperate radially with an edge of a second anastomosis aperture inside the second membrane of the patient's heart,
- the inlet conduct and the outlet conduct may be expandable,
- the inlet conduct and the outlet conduct may present, when expanded, a shape set by radial expansion forces,
- the device may be configured to enable a blood flow of the patient to artificially circulate through a section ranging from 50mm² to 700mm² inside the patient's heart,
- the pump housing may comprise a pump chamber, and wherein the blood pump comprises a motor and an impeller, the pump chamber being configured to surround the impeller and being further configured to connect the inlet conduct and the outlet conduct,
- the inlet conduct may be configured to extend in line with the pump housing.

Another object of the present invention is about an implantation method for a left cardiac assisting device as described here above, the method includes following steps:
- transpiercing the first membrane to create a first anastomosis aperture,
- introducing the removable pump support element in the patient's heart and securing the removable pump support element to the first anastomosis aperture,
- introducing a catheter comprising the folded assisting device inside the patient's heart,
- transpiercing the second membrane to create a second anastomosis aperture,
- securing the outlet conduct to the second anastomosis aperture,
- retrieving the catheter in order to let the outlet conduct expand inside the second anastomosis aperture,
- guiding the inlet conduct through the removable pump support element inside the first anastomosis aperture,
- further retrieving the catheter in order to let the inlet conduct and the pump housing expand inside the patient's heart.

The steps of introducing a catheter comprising the folded assisting device inside the patient's heart, of transpiercing the second membrane to create a second anastomosis aperture, of securing the outlet conduct to the second anastomosis aperture, may all happen simultaneously. The removable pump support element may be retrieved from the patient's heart once the assisting device is secured to the first and second membranes and fully expanded.

### BRIEF DESCRIPTION OF THE DRAWINGS

The invention will be better understood, and other aims, details, characteristics and advantages thereof will emerge more clearly on reading the detailed description which follows, of one or several embodiments of the invention given by way of illustration. Those are purely illustrative and non-limiting examples, with reference to the accompanying schematic drawings. On these drawings:
- **figure 1** is a schematical open view of a classic human heart,
- **figures 2a** schematical perspective view of a classic human heart showing the transverse section planes of figures 2b and 2c,
- **figure 2b** is a schematical transverse sectional view of a classic human heart on a first section plane,
- **figure 2c** is a schematical transverse sectional view of a classic human heart on a second section plane,
- **figure 3a** is the schematical sectional view of figure 2a presenting the second anastomosis according to the present invention,
- **figure 3b** is the schematical sectional view of figure 2b presenting the first anastomosis according to the present invention,
- **figure 4** is a perspective view of an assisting device with exception to the pump support element 20, according to the present invention and its localization inside a patient's heart,
- **figures 5a to 5g** is a series of schematical perspective views of the different steps of the implantation method according to the present invention,
- **figures 6a to 6c** is a series of detailed depictions of the steps taking place in figures 5g, 5h and 5i of the method of the present invention.

### DETAILED DESCRIPTION

### Assisting Device

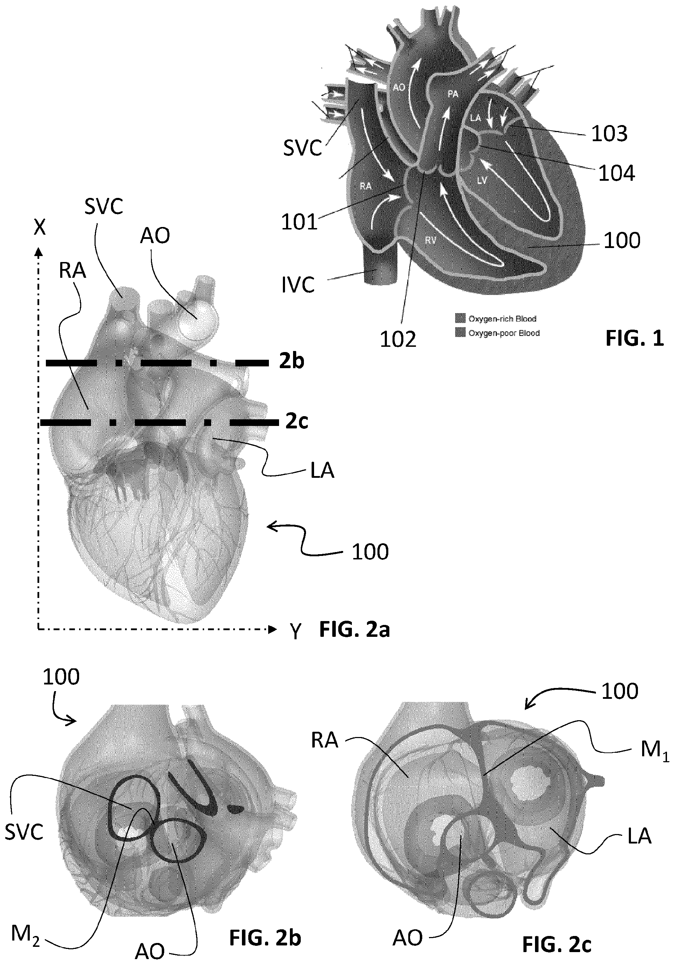

As can be seen on figure 1, a classic human heart 100 presents a long heart axis X and a short heart axis Y. Considering a classic human heart 100, oxygen-poor blood enters the heart 100 through the inferior vena cava IVC or the superior vena cava SVC towards the right atrium RA, crosses the tricuspid valve 101, enters the right ventricle RV, crosses the pulmonary valve 102 into the pulmonary arteries PA and moves on to the lungs. Oxygen-rich blood comes back from the lungs into the heart 100 through the left atrium LA, crosses the mitral valve 103, enters the left ventricle LV, eventually crosses the aortic valve 104 and leaves the heart through the aorta AO.

As already mentioned, here-above, this invention relates to a left cardiac assisting device 10 configured to be implanted inside a patient's heart 100. More particularly, the assisting device 10 according to the present invention is configured to be placed inside the right atrium RA or the superior vena cava SVC of a patient's heart 100.

In order to do so, the assisting device 10 comprises:
- a preferably expandable inlet conduct 12 configured to be secured to a first membrane M₁ the patient's heart 100, this first membrane M1 being preferably the membrane separating either the left atrium LA from the right atrium RA or the left atrium from the superior vena cava SVC,
- a preferably expandable outlet conduct 14 configured to be secured to a second membrane M₂ of the patient's heart 100, the second membrane M₂ separating either the right atrium RA from the aorta AO or the superior vena cava SVC from the aorta AO,
- an expandable or rigid pump housing 16 configured to surround a blood pump 18 and to connect the inside of the inlet conduct 12 to the inside of the outlet conduct 14 in order to create one continuous tube or conduct through which fluid can circulate,
- a removable pump support element 20 configured to cooperate with the inlet conduct 12. More precisely, the removable pump support element 20 is configured to secure and stabilize the implantation of the inlet conduct 12 into the first membrane M₁.

The inlet conduct 12 presents a general tubular shape with a diameter ranging from 8 to 30mm. More precisely, the inlet conduct 12 presents a general cylindrical or conical shape. In the embodiment in which the inlet conduct is expandable, the expandable inlet conduct 12 is made of an expandable material, for example a meshed material. This meshed material could be a metallic or polymer mesh, or a mesh at least partially made out of Nitinol or similar material The material is preferably a selfexpandable material like nitinol or any other similar material. Once implanted inside the patient's heart 100 and once fully expanded, the inlet conduct 12 presents a shape defined by radial expansion forces. The shape of the fully expanded and implanted inlet conduct 12 is also defined by its surroundings, in particular the first membrane M₁, the left atrium LA and the right atrium RA of the patient's heart 100 to which it is secured and through or in which the inlet conduct 12 can expand. The shape of the fully expanded and implanted inlet conduct 12 is therefore defined by the space its surroundings offers. It would thus present a smaller diameter in places in which it cannot expand a lot, for example inside the first membrane M1, and would present a larger diameter in places in which it can expand more, for example in the left atrium LA and the right atrium RA.

The inlet conduct 12 can be secured to the membrane separating the left atrium LA from the right atrium RA of the patient's heart 100. In some other embodiment, the inlet conduct 12 can be secured to the membrane separating the left atrium LA from the superior vena cava SVC of the patient's heart 100. The first membrane M₁ is thus either the membrane separating the left atrium LA from the right atrium RA or the membrane separating the left atrium LA from the superior vena cava SVC of the patient's heart 100.

In order to lead the patient's blood flow inside the assisting device 10, the inlet conduct 12 is secured to the first membrane M₁ by means of a first anastomosis A₁.

In anatomy (and surgery), an anastomosis is commonly known as a connection between two body channels together, such as blood vessels or parts of the intestines. In some cases, surgeons create a new anastomosis after removing or bypassing part of a channel, or after removing or replacing an organ that was connected to a channel.

In the present case, the inlet conduct 12 crosses the first membrane M₁ by means of an artificial first anastomosis A₁ created preferably percutaneously by a surgeon or interventional cardiologist. Said first anastomosis A₁ thus presents an aperture 201 ranging from 8 to 15mm connecting the left atrium LA to the right atrium RA or to the superior vena cava SVC of the patient's heart 100. The aperture 201 is circumscribed by an edge presenting the regular elasticity and width of the membrane M₁ of the patient's heart 100.

The inlet conduct 12 is thus configured to cooperate radially with the edge of the aperture 201 of the first anastomosis A₁ inside the first membrane M₁. This radial cooperation ensures a tight and strong cooperation between the first membrane M₁ and the inlet conduct 12. This radial cooperation thus ensures that the complete blood flow of the patient's heart 100 flows through the first membrane M₁, from the left atrium LA, inside the assisting device 10. In order to optimize the cooperation with the edge of the aperture 201 of the first anastomosis A₁, the inlet conduct 12 can present, on its circumference, a reinforced zone with a distal and a proximal extremity. More precisely, the reinforced zone presents a larger diameter than the diameter of the rest of the inlet conduct 12 in order to tighten the cooperation with the first membrane M₁. The reinforced zone can also present and a disc at its distal extremity (left atrial LA extremity) to avoid migration. It can further present another disc on its proximal extremity thus presenting a double disc shape. Thanks to those discs, the inlet conduct 12 and the membrane M₁ further cooperate by axial abutment. The reinforced zone can be removable.

Similarly to the inlet conduct 12, the outlet conduct 14 presents a general tubular shape. In the embodiments in which the outlet conduct 14 is expandable, the expandable outlet conduct 14 is made of an (auto or balloon) expandable material, for example a meshed material. This meshed material could be a metallic or polymer mesh, or a mesh at least partially made out of Nitinol or similar material. The material is preferably a selfexpandable material like nitinol or any other similar material. The outlet conduct 14 presents a general cylindrical or conical shape with a diameter ranging from 8 to 30mm. Once implanted inside the patient's heart 100 and once fully expanded, the outlet conduct 14 also presents a shape defined by radial expansion forces. The shape of the fully expanded and implanted outlet conduct 14 is also defined by its surroundings, in particular the second membrane M₂ of the patient's heart 100 to which it is secured.

Similarly to the inlet conduct 12, the outlet conduct 14 is secured to the second membrane M₂ by means of a second anastomosis A₂. This enables the patient's blood flow to exit the assisting device 10 and to flow inside the aorta AO of the patient's heart 100. The outlet conduct 14 crosses the second membrane M₂ by means of an artificial second anastomosis A₂ created percutaneously by a surgeon or an interventional cardiologist. Said second anastomosis A₂ presents an aperture 202 with a diameter ranging from 8 to 15mm, connecting the right atrium RA or superior vena cava SVC to the aorta AO of the patient's heart 100. The aperture 202 is also circumscribed by an edge presenting the regular elasticity and width of the membrane M₂ of the patient's heart 100.

The outlet conduct 14 is thus configured to cooperate radially with the edge of the aperture 202 of the second anastomosis A₂ inside the second membrane M₂. This radial cooperation ensures a tight and strong cooperation between the second membrane M₂ and the outlet conduct 14. This radial cooperation thus ensures that the complete blood flow of the patient's heart 100 flows through the second membrane M₂, from the inside of the assisting device 10 inside the aorta AO of the patient's heart 100. In order to optimize the cooperation with the edge of the aperture 202 of the second anastomosis A₂, the outlet conduct 14 can present, on its circumference, a reinforced zone with a distal and a proximal extremity. More precisely, the reinforced zone presents a larger diameter than the diameter of the first anastomosis A₂ aperture. The reinforced zone can also present and a disc at its distal extremity to avoid migration. It can further present another disc on its proximal extremity thus presenting a double disc shape. Thanks to those discs, the outlet conduct 14 and the membrane M₂ further cooperate by axial abutment. The reinforced zone can be removable.

As already mentioned, the expandable or rigid pump housing 16 connects the inlet conduct 12 to the outlet conduct 14. In this context, "connects" means "putting in communication", more precisely putting in communication in order to constitute a continuous channel forming one single functional piece, thus enabling a fluid to circulate from the inlet conduct 12 towards the outlet conduct 14 through the pump housing 16. In order to enable the patient's blood flow to be driven from the inlet conduct 12 to the outlet conduct 14 through the first and second membranes M₁, M₂, of the patient's heart 100, the pump housing 16 presents a global tubular shape and is tightly connected to both the inlet and the outlet conduct 12, 14. The inlet conduct 12, the outlet conduct 14 and the pump housing 16 form one single technical piece. The technical advantage of a device 10 with an inlet conduct 12, an outlet conduct 14 and a pump housing 16 forming one single technical piece, is a greater simplicity and accuracy of implantation. If all those elements were independent from each other, an assembling step should be carried out inside the patient's heart 100 and this would add time and complexity to the intervention, thus raising the surgical risks.

The pump housing 16 is made of the same material than the inlet conduct 12. The pump housing 16 presents a diameter ranging from 6 to 15mm, preferably 10mm.

The pump housing 16 is further designed to be located inside the right atrium RA or the superior vena cava SVC of the patient's heart 100.

In order to put the blood flow in motion through the assisting device 10 once it is implanted inside the patient's heart 100, the blood pump 18 comprises a motor and an impeller. The impeller is magnetically coupled to the motor. The blood pump 18 provides a fluidic pressure ranging from 70 to 130mmhg and a fluidic debit ranging from 2,5 to 3,5L/min. The impeller of the blood pump 18 is comprised within a pump chamber of the pump housing 16. This pump chamber also enables the connection of the inlet conduct 12 to the outlet conduct 14, permitting a fluidic communication from the inlet conduct 12 to the outlet conduct 14 through the pump chamber.

Despite being tightly and strongly connected to each other and forming one single technical piece, in the embodiments in which at least one of the elements of the device 10 is expandable, the inlet conduct 12, the outlet conduct 14 and the pump housing 16 are all three configured to expand independently from each other. This way, for example, the outlet conduct 14 can be expanded while both the inlet conduct 12 and pump housing 16 are still folded. The outlet conduct could also still be folded while both the inlet conduct 12 and pump housing 16 are already expanded. This is valid for any possible configuration of those three elements.

Once implanted, the assisting device 10 thus leads the blood flow of the patient from the left atrium LA through the first membrane M₁ to either the right atrium RA or the superior vena cava SVC. Depending on the embodiments, the blood flow of the patient is then led from either the right atrium RA or the superior vena cava SVC, through the second membrane M₂, to the aorta AO of the patient. This way, the main part of the blood pump 18 of the assisting device 10 is advantageously housed in the right trial position and it is avoided that the blood pump 18 is housed inside the left ventricle LV or left atrium LA which could present several hemodynamic and anatomic constraints. Those constraints could lead to a necessity to select the patients for implantation. Those constraints could also damage the blood pump 18 or the device 10 more generally once implanted. In addition, the implantation is feasible percutaneously by the femoral vein.

The device 10 according to the present invention enables a blood flow of a patient to artificially circulate through a section ranging from 50mm² to 700mm² inside the patient's heart 100. This enables to avoid damaged or weak sections of the patient's heart 100.

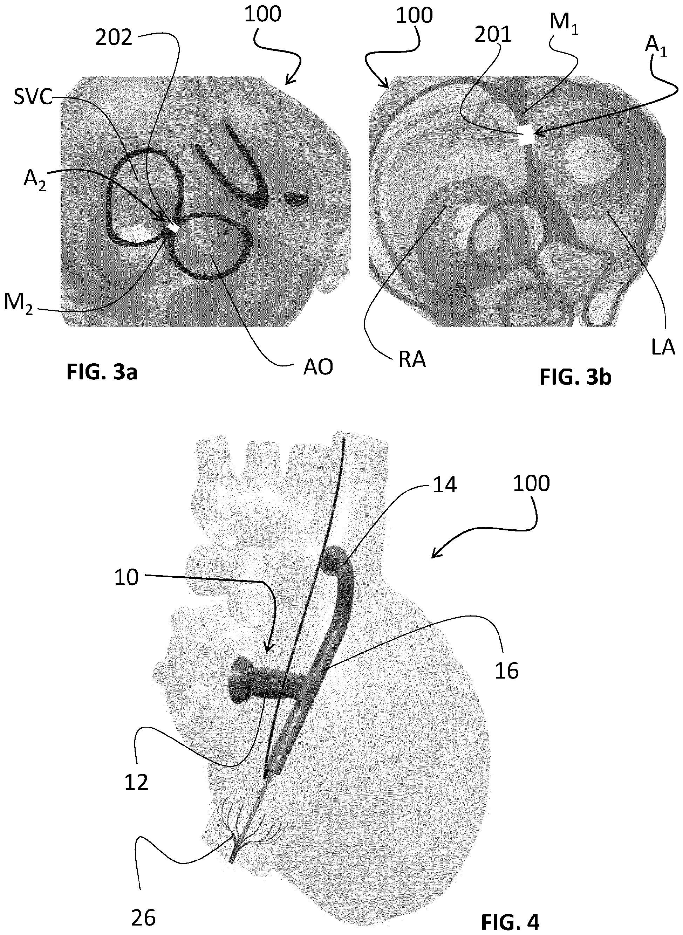

The assisting device 10 according to the present invention is thus configured to extend along the long heart axis X, as can be seen on figure 3. After implantation the inlet conduct 12 is thus configured to be located lower than the outlet 14 conduct regarding said long heart axis X. In order to enable the assisting device 10 to extend along the long heart axis X while crossing the second membrane M₂, the outlet conduct 14 is configured to extend transversally to the long heart axis X. In order to enable the assisting device 10 to extend along the long heart axis X while crossing the first membrane M₁, the inlet conduct 12 is preferably configured to extend in line with the pump housing 16. The assisting device 10 thus presents a general Y or T shape. This specific shape enables to safely house the blood pump 18 inside the long straight part of the Y or T while enabling the device 10 to be implanted percutaneously inside the patient's heart 100 and presenting sufficient space in the short straight parts of the Y or T for conveying a sufficient blood flow (around 40 to 70% of the total blood flow) of the patient from the left atrium to the aorta in parallel to the native way through the left ventricle.

As already mentioned here-above, the assisting device 10 according to the present invention further comprises a removable pump support element 20. This removable pump support element 20 is configured to enable the guiding of the inlet conduct 12 through the first membrane M1 of the patient's heart during implantation. More precisely, the function of the removable pump support element 20 is to lead the inlet conduct 12 to its right position inside the left atrium LA. Thus, the removable pump support element 20 is to stabilize the expansion position of the inlet conduct 12 in the left atrium LA. A further function of the removable pump support element 20 is to avoid a move back of the inlet conduct 12 into the right atrium RA or the superior vena cava SVC.

More precisely, the removable pump support element 20 is configured to be removably secured to the first membrane M₁ of the patient's heart 100. More precisely, the removable pump support element 20 is configured to be removably secured to the first membrane M₁ by means of the first anastomosis A₁. The removable pump support element 20 thus presents a tubular shape configured to radially cooperate with the edge of the aperture 201 of the first anastomosis A₁.

In some embodiments, the removable pump support element 20 is removed after the inlet conduct 12 has been placed inside the first anastomosis A₁ of the first membrane M₁. In some other embodiments, the removable support element 20 is not removed.

The removable pump support 20 presents the shape of a very small catheter. It presents the shape of a tube with a diameter ranging from 1 to 7mm, preferably 3mm. The removable pump support 20 comprises an internal guide, configured to cooperate with the inlet conduct 12. The removable pump support 20 is rigid enough to guide and support the inlet conduct 12 to be secured in the right position inside the patient's heart 100. In order to enable its insertion in the first anastomosis A₁, the inlet conduct 12 presents a bendable and flexible end. Once bent, the removable pump support 20 thus forms a general L shape.

This L shape ensures the safe placement of the inlet conduct 12 through the first membrane M₁, inside the left atrium LA as it enables three bearing or attachment points, while enabling its insertion in a very small and narrow part of the patient's heart 100.

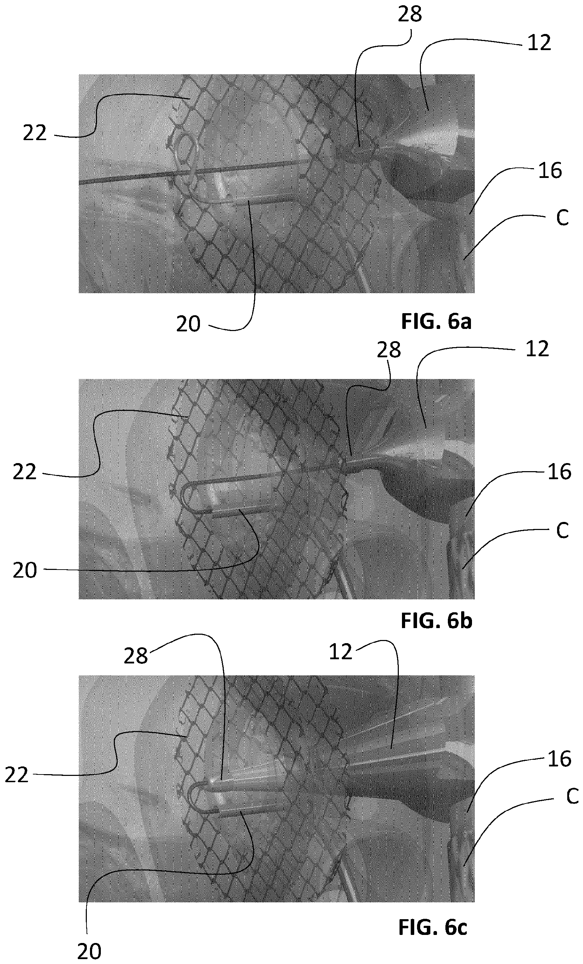

The removable pump support element 20 is technically relevant for the implantation of the assisting device 10, as can be seen on figures 6a to 6c, in order to enable a precise positioning of the inlet conduct 12 of the device, without the need of introducing unnecessary tools. In a very small and narrow part of the human heart 100.

In some embodiments, the device 10 further comprises a first anti-reflux stent 22 configured to be secured inside the aperture 201 of the first anastomosis A₁. In those embodiments, the inlet conduct 12 is secured to the first anti-reflux stent 22, inside the aperture 201. The first anti-reflux stent 22 is configured to avoid any fluidic circulation from the outlet conduct 14 towards the inlet conduct 12. It might for example comprise an anti-reflux valve. The first anti-reflux stent 22 can form a removable reinforced zone of the inlet conduct 12.

In some embodiments, the device 10 further comprises a second anti-reflux stent 24 configured to be secured inside the aperture 202 of the second anastomosis A₂. In those embodiments, the outlet conduct 14 is secured to the second anti-reflux stent 24, inside the aperture 202. The second anti-reflux stent 24 is configured to avoid any fluidic circulation from the outlet conduct 14 towards the inlet conduct 12. It might for example comprise an anti-reflux valve. The second anti-reflux stent 24 can form a removable reinforced zone of the outlet conduct 14.

In some embodiments, the device 10 further comprises a stabilization element 26 configured to be secured to the wall of the inferior vena cava IVC of the patient's heart 100. This stabilization element could counterbalance the important weight of the motor inside the device 10.

### Implantation method

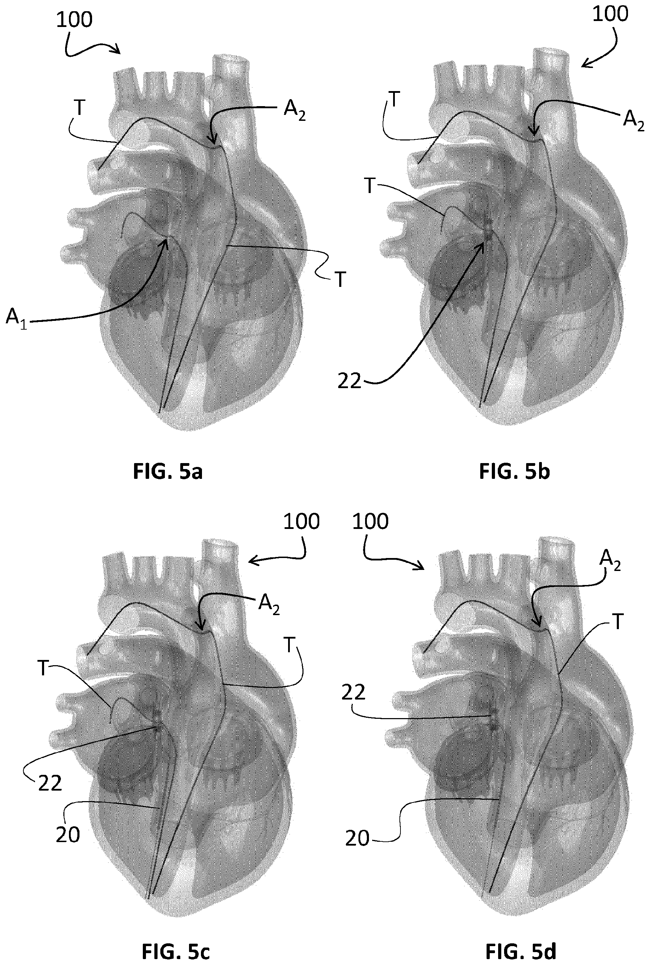

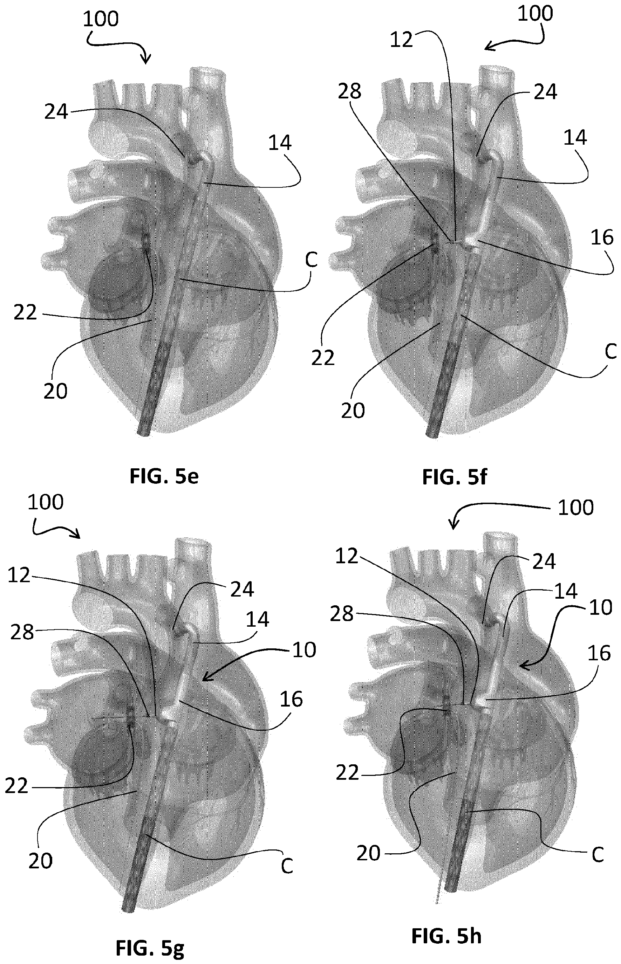

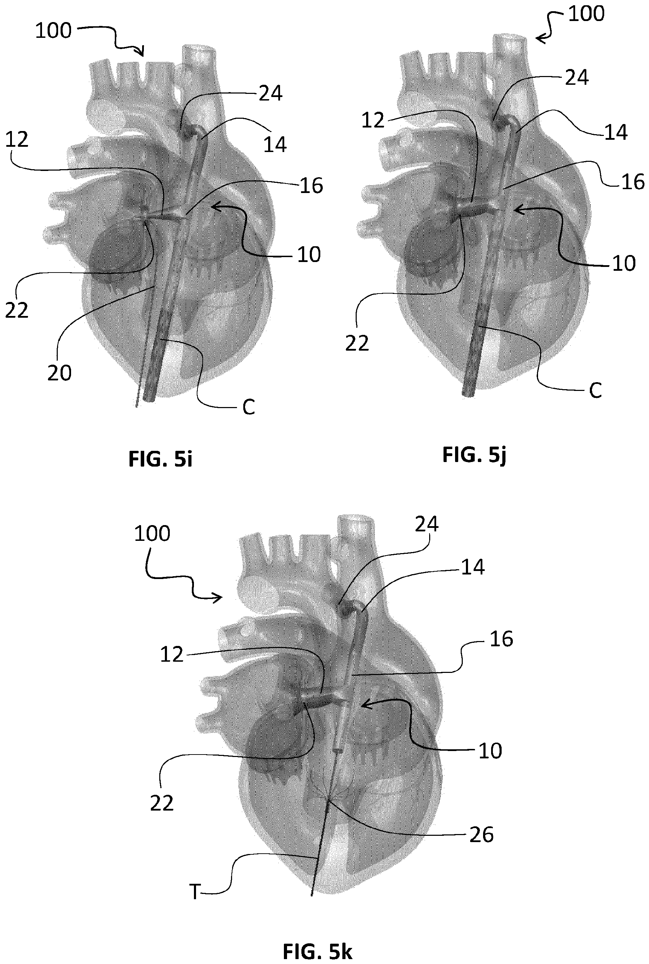

In order to implant the left assisting device 10 into a patient's heart 100, a possible implantation method is carried out according to the following steps:
- inserting inside the patient's heart 100 at least one transeptal needle T,
- transpiercing, by means of the at least one transeptal needle T the first membrane M₁ to create the first anastomosis A₁ aperture 201 (see figure 5a),
- transpiercing, by means of the at least one transeptal needle T the second membrane M₂ to create a second anastomosis A₂ aperture 202 (see figure 5a),
- if necessary, inserting the first anti-reflux stent 22 inside the aperture 201 of the first anastomosis A₁ (see figure 5b),
- introducing the removable pump support element 20 in the patient's heart 100 and securing the pump support element 20 to the first anastomosis aperture 201 in a removable way (see figure 5c),
- retrieving the transeptal needle T from the first anastomosis A₁ (see figure 5d),
- introducing a catheter C comprising the inlet conduct 12, outlet conduct 14 and pump body 16 of the assisting device 10, preferably folded, inside the patient's heart 100,
- if not already transpierced, transpiercing the second membrane M₂ to create a second anastomosis A₂ aperture 202,
- if necessary, inserting the second anti-reflux stent 24 inside the aperture 202 in order to avoid any blood flow back from the aorta AO to the superior vena cava SVC,
- securing the outlet conduct 14 to the second anastomosis A₂ aperture 202,
- retrieving the catheter C in order to let the outlet conduct 14 expand inside the second anastomosis aperture 202 (see figure 5e),
- retrieving the catheter C in order to let at least a part of the pump body 16 expand inside the patient's heart 100 (see figure 5f),
- guiding the inlet conduct 12 by means of the removable pump support element 20 inside the first anastomosis aperture 201 (see figures 5g to 5j and 6a to 6c),
- further retrieving the catheter C in order to let the inlet conduct 12 and, if necessary, the complete pump housing 16 expand inside the patient's heart 100 (see figure 5k),
- if necessary, the stabilization element 26 is put in place to stabilize the blood pump 18 and contributes to its keeping its correct position.

In the embodiments in which the device presents at least one expandable element, t/he inlet conduct 12, the outlet conduct 14 and/or the pump housing 16 remain folded as long they are contained inside the catheter and expand as soon the catheter is retrieved and they are set free inside the patient's heart 100. Once the inlet conduct 12, outlet conduct 14 and pump housing 16 (if expandable) are freed from the catheter, they expand and adapt to their environment, occupying all the available space, thus radially cooperating with the edges of the apertures 201, 202 of the created anastomosis A₁, A₂ or the heart chambers they are located in. In the embodiments, in which the inlet conduct 12 and/or the outlet conduct 14 and/or the pump housing 16 is rigid, the rigid elements do not change shape when freed from the catheter.

In some embodiments, the inlet conduct wears a protection cap 28 (see figures 5f to 5i) which maintains it folded even outside the catheter C. In those embodiments, the removable pump support element 20 is configured to cooperate with said protection cap 28 to remove it from the inlet conduct 12 once it is well placed inside the patient's heart 100.

Depending on the surgeon and/or the patient, the steps of introducing a catheter comprising the folded assisting device 10 inside the patient's heart 100, of transpiercing the second membrane M₂ to create the second anastomosis aperture 202, of securing the outlet conduct 14 to the second anastomosis aperture 202, all happen simultaneously. This enables to minimize the potential blood leakage.

If necessary, the removable pump support element 20 is retrieved from the patient's heart 100 once the assisting device 10 is secured to the first and second membranes M₁, M₂ and fully expanded.

The specific shapes of the different technical elements of the assisting device 10 according to the present invention, and the specific way of implantation enabled by those specific shapes are innovations which enable to efficiently assist any patient with severe heart failure for a long term in the less possible invasive.

## Claims

1. Left cardiac assisting device (10) configured to be implanted inside a patient's heart (100), said assisting device (10) comprising:
- an inlet conduct (12) configured to be secured to a first membrane (M₁) of the patient's heart (100),
- an outlet conduct (14) configured to be secured to a second membrane (M₂) of the patient's heart (100), the second membrane (M₂) separating either the right atrium (RA) from the aorta (AO) or the superior vena cava (SVC) from the aorta (AO),
- a pump housing (16) configured to surround a blood pump (18), said pump housing (16) connecting the inlet conduct (12) to the outlet conduct (14), the pump housing (16) being further designed to be located inside the right atrium (RA) or the superior vena cava (SVC) of the patient's heart (100),
- a removable pump support element (20) configured to cooperate with the inlet conduct (12),
**wherein**
- the inlet conduct (12), the outlet conduct (14) and the pump housing (16) form one single technical piece, in order to enable the patient's blood flow to be driven from the inlet conduct (12) to the outlet conduct (14) through the first and second membranes (M₁, M₂) the patient's heart (100),
- the inlet conduct (12), the outlet conduct (14) and the pump housing (16) are configured to expand independently from each other,
- the removable pump support element (20) is configured to enable the guiding of the inlet conduct (12) through the first membrane (M₁) of the patient's heart (100) during implantation.

2. Left assisting device (10) according to the preceding claim, **wherein** the assisting device (10) is configured to extend along the long heart axis (X), the inlet conduct (12) being thus configured to be located lower than the outlet conduct (14) regarding said long heart axis (X), inside the patient's heart (100).

3. Left assisting device (10) according to the preceding claim, **wherein** the outlet conduct (14) is configured to extend transversally to the long heart axis (X), inside the patient's heart (100).

4. Left assisting device (10) according to any one of the preceding claims, **wherein** the assisting device (10) presents a general Y or T shape.

5. Left assisting device (10) according to any one of the preceding claims, **wherein** the removable pump support element (20) can be bent in order to present a general L shape and is configured to be removably secured to the first membrane (M₁) of the patient's heart (100).

6. Left assisting device (10) according to any one of the preceding claims, **wherein** the first membrane (M₁) is the membrane separating the left atrium (LA) from the right atrium (RA) of the patient's heart (100), or the membrane separating the left atrium (LA) from the superior vena cava (SVC) from the patient's heart (100).

7. Left assisting device (10) according to any one of the preceding claims, **wherein** the inlet conduct (12) is configured to cooperate radially with an edge of a first anastomosis (A₁) aperture (201) inside the first membrane (M₁), and the outlet conduct (14) is configured to cooperate radially with an edge of a second anastomosis (A₂) aperture (202) inside the second membrane (M₂) of the patient's heart (100).

8. Left assisting device (10) according to any one of the preceding claims, **wherein** the inlet conduct (12) and the outlet conduct (14) are expandable.

9. Left assisting device (10) according to the preceding claim, **wherein** the inlet conduct (12) and the outlet conduct (14) present, when expanded, a shape set by radial expansion forces.

10. Left assisting device (10) according to any one of the preceding claims, **wherein** the device (10) is configured to enable a blood flow of the patient to artificially circulate through a section ranging from 50mm² to 700mm² inside the patient's heart (100).

11. Left assisting device (10) according to any one of the preceding claims, **wherein** the pump housing (16) comprises a pump chamber, and wherein the blood pump (18) comprises a motor and an impeller, the pump chamber being configured to surround the impeller and being further configured to connect the inlet conduct (12) and the outlet conduct (14).

12. Left assisting device (10) according to any one of the preceding claims, **wherein** the inlet conduct (12) is configured to extend in line with the pump housing (16).

13. Implantation method for a left cardiac assisting device (10) according to any one of the preceding claims, **wherein** the method includes following steps:
- transpiercing the first membrane (M₁) to create a first anastomosis (A₁) aperture (201),
- introducing the removable pump support element (20) in the patient's heart (100) and securing the removable pump support element (20) to the first anastomosis (A₁) aperture (201),
- introducing a catheter comprising the folded assisting device (10) inside the patient's heart (100),
- transpiercing the second membrane (M₂) to create a second anastomosis (A₂) aperture (202),
- securing the outlet conduct (14) to the second anastomosis (A₂) aperture (202),
- retrieving the catheter in order to let the outlet conduct (14) expand inside the second anastomosis (A₂) aperture (202),
- guiding the inlet conduct (12) through the removable pump support element (20) inside the first anastomosis (A₁) aperture (201),
- further retrieving the catheter in order to let the inlet conduct (12) and the pump housing (16) expand inside the patient's heart (100).

14. Implantation method according to the preceding claim, **wherein** the steps of introducing a catheter comprising the folded assisting device (10) inside the patient's heart (100), of transpiercing the second membrane (M₂) to create a second anastomosis (A₂) aperture (202), of securing the outlet conduct (14) to the second anastomosis (A₂) aperture (202), all happen simultaneously.

15. Implantation method according to claims **13** and **14**, **wherein** the removable pump support element (20) is retrieved from the patient's heart (100) once the assisting device (10) is secured to the first and second membranes (M₁, M₂) and fully expanded.
